# EUROPÄISCHE PATENTSCHRIFT

(11) **EP 2 503 986 B1**
(45) Veröffentlichungstag und Bekanntmachung des Hinweises auf die Patenterteilung: **09.09.2015**
(21) Anmeldenummer: 10781899.9
(22) Anmeldetag: 23.11.2010
(51) Int. Cl.: A61K 8/84, C08G 64/02, C08G 64/42

(54) **VERWENDUNG VON HOCHVERZWEIGTEN POLYCARBONATEN IN KOSMETISCHEN UND DERMATOLOGISCHEN FORMULIERUNGEN**
USE OF HYPERBRANCHED POLYCARBONATES IN COSMETIC AND DERMATOLOGICAL FORMULATIONS
UTILISATION DE POLYCARBONATES TRÈS RAMIFIÉS DANS DES FORMULATIONS COSMÉTIQUES ET DERMATOLOGIQUES

(30) Priorität: 26.11.2009 EP 09177203
(43) Veröffentlichungstag der Anmeldung: 03.10.2012
(73) Patentinhaber: BASF SE, 67056 Ludwigshafen (DE)
(72) Erfinder: WENDEL, Volker, 64342 Seeheim-Jugenheim (DE); LAUBENDER, Matthias, 67105 Schifferstadt (DE); STUMBE, Jean-Francois, F-67200 Strasbourg (FR); BRUCHMANN, Bernd, 67251 Freinsheim (DE)
(86) Internationale Anmeldenummer: PCT/EP2010/067982
(87) Internationale Veröffentlichungsnummer: WO 2011/064187

(56) Entgegenhaltungen:
- WO-A1-2008/012252
- WO-A1-2010/130599
- WO-A2-2007/135032
- WO-A2-2010/145993
- US-A1- 2008 153 931
- US-B1- 6 475 495

## Beschreibung

Die vorliegende Erfindung betrifft die Verwendung von substituierten hochverzweigten Polycarbonate als Verdicker in der Kosmetik und der Dermatologie.

Verdickungsmittel werden auf dem Gebiet der Pharmazie und der Kosmetik in großem Umfang zur Erhöhung der Viskosität von Zubereitungen eingesetzt.

Das Verdickungsmittel wird danach ausgewählt, ob die Zubereitung wässrig, ölig oder tensidisch ist. Eine Übersicht hierzu gibt Hugo Janistyn, Handbuch der Kosmetika und Riechstoffe, Hüthig Verlag Heidelberg, Band 1, 3. Auflage, 1978, S. 979.

Beispiele für häufig verwendete Verdickungsmittel für wässrige Lösungen sind Fettsäurepolyethylenglycolmonoester, Fettsäurepolyethylenglycoldiester, Fettsäurealkanolamide, oxethylierte Fettalkohole, ethoxylierte Glycerinfettsäureester, Celluloseether, Natriumalginat, Polyacrylsäuren sowie Neutralsalze.

Auch Carboxylgruppen enthaltende Polymere sind als Verdicker bekannt. Dazu zählen Homo- und Copolymere von monoethylenisch ungesättigten Carbonsäuren wie Acrylsäure, Methacrylsäure, Maleinsäure, Maleinsäureanhydrid und Itaconsäure. Diese Polymere sind häufig wenigstens zu einem geringen Anteil vernetzt. Solche Polymere sind beispielsweise in US 2,798,053, US 3,915,921, US 3,940,351 , US 4,062,817, US 4,066,583, US 4,267,103, US 5,349,030 und US 5,373,044 beschrieben.

Häufige Nachteile dieser Polymere bei der Verwendung als Verdicker sind deren pH-Abhängigkeit und hydrolytische Instabilität. Weiterhin werden häufig große Mengen der Polymere benötigt, um den erwünschten Verdickungs-Effekt zu erzielen und die Stabilität der Zubereitungen bei Anwesenheit von Elektrolyten ist gering.

Auch natürlich vorkommende Materialien wie Casein, Alginate, Methylcellulose, Hydroxyethylcellulose, Hydroxypropylcellulose und Carbomethoxycellulose werden als Verdicker eingesetzt. Diese haben u.a. den Nachteil der Empfindlichkeit gegenüber mikrobiologischen Faktoren und es bedarf folglich des Zusatzes von Bioziden.

Typische Verdicker von öligen Zubereitungen, im folgenden auch Ölverdicker genannt, sind Metallseifen, amorphes Siliciumdioxid, Hydroxystearin, Verbindungen quaternärer Ammoniumbasen mit Bentoniten, Wachse und Paraffine.

Tensidlösungen werden beispielsweise verdickt durch Fettsäurealkylolamide, Aminoxide, Cellulosederivate, Polysaccharide und die oben genannten Carboxylgruppen enthaltende Polymere.

Eine Aufgabe der vorliegenden Erfindung war es, verdickende, insbesondere ölverdickende Polymere zu finden, die für kosmetische Anwendungen gut geeignet sind und insbesondere im Gebiet Hautkosmetik gute anwendungstechnische Eigenschaften besitzen. Dazu gehören neben der guten Verdickerwirkung bei geringem Materialeinsatz auch Klarheit bei Gelanwendungen, (co-) emulgierende und stabilisierende Wirkung für ölunlösliche und/oder schwer zu stabilisierende Komponenten, gute Einarbeitbarkeit in kosmetische Zubereitungen. Insbesondere für Gele ist eine möglichst hohe Transparenz (Klarheit) der Zubereitungen erwünscht. Um eine breitestmögliche Formulierbarkeit zu gewährleisten ist es erwünscht, dass die Verdicker farb- und geruchsarm, idealerweise farb- und geruchslos sind.

Zudem ist es bei der Verwendung in (haut)kosmetischen und/oder dermatologischen Anwendungen notwendig, dass keine allergenen Reaktionen ausgelöst werden.

Die Aufgabe wird durch die nachstehend beschriebenen hochverzweigten Polycarbonate gelöst.

Die Herstellung hochfunktioneller hochverzweigter Polycarbonate und deren Verwendung als Haftvermittler, Thixotropiermittel oder als Bausteine zur Herstellung von Polyadditions- oder Polykondensationspolymeren, zum Beispiel von Lacken, Überzügen, Klebstoffen, Dichtmassen, Gießelastomeren oder Schaumstoffen ist aus der WO 2005/026234 bekannt.

WO 2006/018063 beschreibt Zusammensetzungen für die Haarkosmetik, die hydrophob funktionalisierte dendritische Makromoleküle enthalten. Die dendritischen Makromoleküle sind entweder aus Polyestereinheiten (erhältlich unter dem Handelsnamen Boltorn) oder aus Polyamideinheiten (erhältlich unter dem Handelsnamen Hybrane) aufgebaut.

DE 10 2005 063 096 beschreibt kosmetische Mittel, die 0,05 bis 20 Gew.-% mindestens eines hyperverzweigten Polyesters und/oder Polyesteramides enthalten. Die Mittel sollen haarreinigende und/oder haarpflegende Eigenschaften aufweisen. Die Polyester und/oder Polyesteramide sind nicht substituiert.

In der WO 2004/078809 werden hochverzweigte Polymere und diese enthaltende kosmetische Zusammensetzungen offenbart.

Unter hochverzweigten Polycarbonaten werden im Rahmen dieser Erfindung unvernetzte Makromoleküle mit Hydroxyl- und Carbonat- oder Carbamoylchloridgruppen verstanden, die sowohl strukturell als auch molekular uneinheitlich sind. Sie können auf der einen Seite ausgehend von einem Zentralmolekül analog zu Dendrimeren, jedoch mit uneinheitlicher Kettenlänge der Äste aufgebaut sein. Sie können auf der anderen Seite auch linear, mit funktionellen Seitengruppen, aufgebaut sein oder aber, als Kombination der beiden Extreme, lineare und verzweigte Molekülteile aufweisen. Zur Definition von dendrimeren und hochverzweigten Polymeren siehe auch P.J. Flory, J. Am. Chem. Soc. 1952, 74, 2718 und H. Frey et al., Chem. Eur. J. 2000, 6, No. 14, 2499.

Unter "hochverzweigt" wird im Zusammenhang mit der vorliegenden Erfindung verstanden, dass der Verzweigungsgrad (Degree of Branching, DB), dass heißt die mittlere Anzahl dendritischer Verknüpfungen plus mittlere Anzahl der Endgruppen pro Molekül geteilt durch die Summe der mittleren Anzahl der dendritischen Verknüpfungen, der mittleren Anzahl der linearen Verknüpfungen und der mittleren Anzahl der Endgruppen, multipliziert mit 100 , 10 bis 99.9 %, bevorzugt 20 bis 99 %, besonders bevorzugt 20 - 95 % beträgt.

Aus der Literatur ist neben dem Ausdruck hochverzweigt auch der Ausdruck hyperverzweigt bekannt. Im Rahmen der vorliegenden Erfindung sollen die beiden Ausdrücke synonym verstanden werden.

Unter "dendrimer" wird im Zusammenhang mit der vorliegenden Erfindung verstanden, daß der Verzweigungsgrad 99,9 - 100% beträgt. Zur Definition des "Degree of Branching" siehe H. Frey et al., Acta Polym. 1997, 48, 30.

Die hochverzweigten Polycarbonate werden wie nachfolgend beschrieben hergestellt.

Als Ausgangsmaterial kann Phosgen, Diphosgen oder Triphosgen eingesetzt werden, vorzugsweise werden jedoch organische Carbonate (A) verwendet.

Bei den Resten R der als Ausgangsmaterial eingesetzten organischen Carbonate (A) der allgemeinen Formel RO[(CO)O]ₙR handelt es sich jeweils unabhängig voneinander um einen geradkettigen oder verzweigten aliphatischen, aromatisch/aliphatischen oder aromatischen Kohlenwasserstoffrest mit 1 bis 20 C-Atomen handelt. Die beiden Reste R können auch unter Bildung eines Ringes miteinander verbunden sein. Bevorzugt handelt es sich um einen aliphatischen Kohlenwasserstoffrest und besonders bevorzugt um einen geradkettigen oder verzweigten Alkylrest mit 1 bis 5 C-Atomen, oder um einen substituierten oder unsubstituierten Phenylrest.

Bei den Carbonaten kann es sich bevorzugt um einfache Carbonate der allgemeinen Formel RO(CO)OR handeln, d.h. in diesem Falle steht n für 1.

Generell handelt es sich bei n um eine ganze Zahl zwischen 1 und 5, bevorzugt zwischen 1 und 3.

Dialkyl- oder Diarylcarbonate können zum Beispiel hergestellt werden aus der Reaktion von aliphatischen, araliphatischen oder aromatischen Alkoholen, vorzugsweise Monoalkoholen mit Phosgen. Weiterhin können sie auch über oxidative Carbonylierung der Alkohole oder Phenole mittels CO in Gegenwart von Edelmetallen, Sauerstoff oder NOₓ hergestellt werden. Zu Herstellmethoden von Diaryl- oder Dialkylcarbonaten siehe auch "Ullmann's Encyclopedia of Industrial Chemistry", 6th Edition, 2000 Electronic Release, Verlag Wiley-VCH.

Beispiele geeigneter Carbonate umfassen aliphatische, aromatisch/aliphatische oder aromatische Carbonate wie Ethylencarbonat, 1,2- oder 1,3-Propylencarbonat, Diphenylcarbonat, Ditolylcarbonat, Dixylylcarbonat, Dinaphthylcarbonat, Ethylphenylcarbonat, Dibenzylcarbonat, Dimethylcarbonat, Diethylcarbonat, Dipropylcarbonat, Dibutylcarbonat, Diisobutylcarbonat, Dipentylcarbonat, Dihexylcarbonat, Dicyclohexylcarbonat, Diheptylcarbonat, Dioctylcarbonat, Didecylacarbonat oder Didodecylcarbonat.

Beispiele für Carbonate, bei denen n größer als 1 ist, umfassen Dialkyldicarbonate, wie Di(tert.butyl)dicarbonat oder Dialkyltricarbonate wie Di(tert.butyl)tricarbonat.

Bevorzugt werden aliphatische Carbonate eingesetzt, insbesondere solche, bei denen die Reste 1 bis 5 C-Atome umfassen, wie zum Beispiel Dimethylcarbonat, Diethylcarbonat, Dipropylcarbonat, Dibutylcarbonat oder Diisobutylcarbonat oder Diphenylcarbonat als aromatisches Carbonat.

Die organischen Carbonate werden mit mindestens einem aliphatischen oder aromatischen Alkohol (B), welcher mindestens 3 OH-Gruppen aufweist oder Gemischen zweier oder mehrerer verschiedener Alkohole umgesetzt.

Beispiele für Verbindungen mit mindestens drei OH-Gruppen umfassen Glycerin, Trimethylolmethan, Trimethylolethan, Trimethylolpropan, 1,2,4-Butantriol, Tris(hydroxymethyl)amin, Tris(hydroxyethyl)amin, Tris(hydroxypropyl)amin, Pentaerythrit, Diglycerin, Triglycerin, Polyglycerine, Bis(tri-methylolpropan), Tris(hydroxymethyl)isocyanurat, Tris(hydroxyethyl)isocyanurat, Phloroglucinol, Trihydroxytoluol, Trihydroxydimethylbenzol, Phloroglucide, Hexahydroxybenzol, 1,3,5-Benzoltrimethanol, 1,1,1-Tris(4'-hydroxy-phenyl)methan, 1,1,1-Tris(4'-hydroxyphenyl)ethan, Zucker, wie zum Beispiel Glucose, Zuckerderivate, tri- oder höherfunktionelle Polyetherole auf Basis tri- oder höherfunktioneller Alkohole und Ethylenoxid, Propylenoxid oder Butylenoxid oder deren Gemischen, oder Polyesterole. Dabei sind Glycerin, Trimethylolethan, Trimethylolpropan, 1,2,4-Butantriol, Pentaerythrit, sowie deren Polyetherole auf Basis von Ethylenoxid oder Propylenoxid besonders bevorzugt.

Diese mehrfunktionellen Alkohole können auch in Mischung mit difunktionellen Alkoholen (B') eingesetzt werden, mit der Maßgabe, dass die mittlere OH-Funktionalität aller eingesetzten Alkohole zusammen größer als 2 ist. Beispiele geeigneter Verbindungen mit zwei OH-Gruppen umfassen Ethylenglykol, Diethylenglykol, Triethylenglykol, 1,2-und 1,3-Propandiol, Dipropylenglykol, Tripropylenglykol, Neopentylglykol, 1,2-, 1,3- und 1,4-Butandiol, 1,2-, 1,3- und 1,5-Pentandiol, Hexandiol, Cyclopentandiol, Cyclohexandiol, Cyclohexandimethanol, Bis(4-Hydroxycyclohexyl)methan, Bis(4-Hydroxycyclohexyl)ethan, 2,2- Bis(4-Hydroxycyclohexyl)propan, 1,1'-Bis(4-Hydroxyphenyl)-3,3-5-trimethylcyclohexan, Resorcin, Hydrochinon, 4,4'-Dihydroxydiphenyl, Bis-(4-Hydroxyphenyl)sulfid, Bis(4-Hydroxyphenyl)sulfon, Bis(hydroxymethyl)benzol, Bis(Hydroxymethyl)toluol, Bis(p-hydroxyphenyl)methan, Bis(p-hydroxyphenyl)ethan, 2,2-Bis(p-hydroxyphenyl)propan, 1,1-Bis(p-hydroxyphenyl)cyclohexan, Dihydroxybenzophenon, difunktionelle Polyetherpolyole auf Basis Ethylenoxid, Propylenoxid, Butylenoxid oder deren Gemische, Polytetrahydrofuran, Polycaprolacton oder Polyesterole auf Basis von Diolen und Dicarbonsäuren.

Die Diole dienen zur Feineinstellung der Eigenschaften des Polycarbonates. Falls difunktionelle Alkohole eingesetzt werden, wird das Verhältnis von difunktionellen Alkoholen (B') zu den mindestens trifunktionellen Alkoholen (B) vom Fachmann je nach den gewünschten Eigenschaften des Polycarbonates festgelegt. Im Regelfalle beträgt die Menge des oder der Alkohole (B') 0 bis 39,9 mol % bezüglich der Gesamtmenge aller Alkohole (B) und (B') zusammen. Bevorzugt beträgt die Menge 0 bis 35 mol %, besonders bevorzugt 0 bis 25 mol % und ganz besonders bevorzugt 0 bis 10 mol %.

Die hochfunktionellen hochverzweigten Polycarbonate sind nach der Reaktion, also ohne weitere Modifikation, mit Hydroxylgruppen und/oder mit Carbonatgruppen beziehungsweise Carbamoylchloridgruppen terminiert. Sie lösen sich gut in verschiedenen Lösemitteln, zum Beispiel in Wasser, Alkoholen, wie Methanol, Ethanol, Butanol, Alkohol/Wasser-Mischungen, Aceton, 2-Butanon, Essigester, Butylacetat, Methoxypropylacetat, Methoxyethylacetat, Tetrahydrofuran, Dimethylformamid, Dimethylacetamid, N-Methylpyrrolidon, Ethylencarbonat oder Propylencarbonat.

Unter einem hochfunktionellen Polycarbonat ist im Rahmen dieser Erfindung ein Produkt zu verstehen, das neben den Carbonatgruppen, die das Polymergerüst bilden, end- oder seitenständig weiterhin mindestens drei, bevorzugt mindestens sechs, mehr bevorzugt mindestens zehn funktionelle Gruppen aufweist. Bei den funktionellen Gruppen handelt es sich um Carbonatgruppen beziehungsweise Carbamoylchloridgruppen und/oder um OH-Gruppen. Die Anzahl der end- oder seitenständigen funktionellen Gruppen ist prinzipiell nach oben nicht beschränkt, jedoch können Produkte mit sehr hoher Anzahl funktioneller Gruppen unerwünschte Eigenschaften, wie beispielsweise hohe Viskosität oder schlechte Löslichkeit, aufweisen. Die hochfunktionellen Polycarbonate der vorliegenden Erfindung weisen zumeist nicht mehr als 500 end- oder seitenständige funktionelle Gruppen, bevorzugt nicht mehr als 100 end oder seitenständige funktionelle Gruppen auf.

Die einfachste Struktur des Kondensationsproduktes (K), dargestellt am Beispiel der Umsetzung eines Carbonats (A) mit einem Di- oder Polyalkohol (B) ergibt dabei die Anordnung XYₘ oder YₘX, wobei X eine Carbonatgruppe, Y eine Hydroxyl-Gruppe und m in der Regel eine ganze Zahl zwischen 1 und 6, vorzugsweise zwischen 1 und 4, besonders bevorzugt zwischen 1 und 3 darstellt. Die reaktive Gruppe, die dabei als einzelne Gruppe resultiert, wird im folgenden generell "fokale Gruppe" genannt.

Liegt beispielsweise bei der Herstellung des einfachsten Kondensationsproduktes (K) aus einem Carbonat und einem zweiwertigen Alkohol das Umsetzungsverhältnis bei 1:1, so resultiert im Mittel ein Molekül des Typs XY, veranschaulicht durch die allgemeine Formel 1.

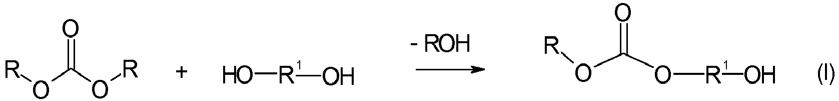

Bei der Herstellung des Kondensationsproduktes (K) aus einem Carbonat und einem dreiwertigen Alkohol bei einem Umsetzungsverhältnis von 1 : 1 resultiert im Mittel ein Molekül des Typs XY₂, veranschaulicht durch die allgemeine Formel 2. Fokale Gruppe ist hier eine Carbonatgruppe.

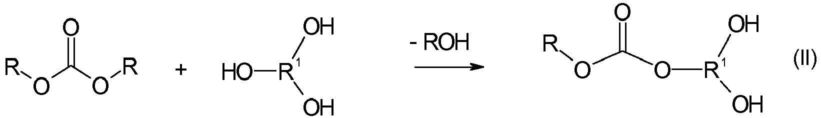

Bei der Herstellung des Kondensationsproduktes (K) aus einem Carbonat und einem vierwertigen Alkohol ebenfalls mit dem Umsetzungsverhältnis 1 : 1 resultiert im Mittel ein Molekül des Typs XY₃, veranschaulicht durch die allgemeine Formel 3. Fokale Gruppe ist hier eine Carbonatgruppe.

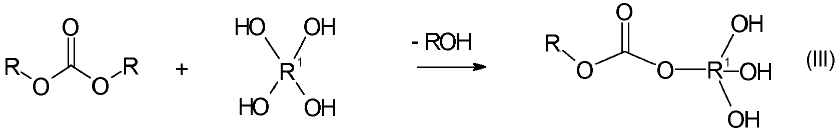

In den Formeln 1 bis 3 hat R die eingangs definierte Bedeutung und R¹steht für einen aliphatischen oder aromatischen Rest.

Weiterhin kann die Herstellung des Kondensationsprodukts (K) zum Beispiel auch aus einem Carbonat und einem dreiwertigen Alkohol, veranschaulicht durch die allgemeine Formel 4, erfolgen, wobei das Umsetzungsverhältnis bei molar 2:1 liegt. Hier resultiert im Mittel ein Molekül des Typs X₂Y, fokale Gruppe ist hier eine OH-Gruppe. In der Formel 4 haben R und R¹ die gleiche Bedeutung wie in den Formeln 1 bis 3.

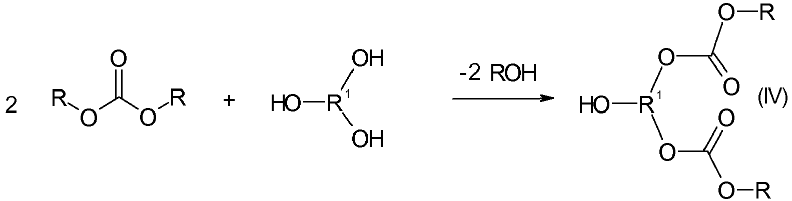

Werden zu den Komponenten zusätzlich difunktionelle Verbindungen, z.B ein Dicarbonat oder ein Diol gegeben, so bewirkt dies eine Verlängerung der Ketten, wie beispielsweise in der allgemeinen Formel 5 veranschaulicht. Es resultiert wieder im Mittel ein Molekül des Typs XY₂, fokale Gruppe ist eine Carbonatgruppe.

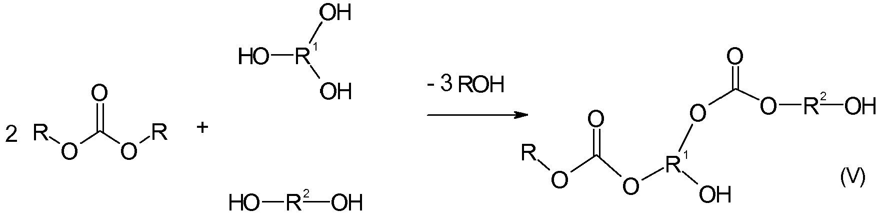

In Formel 5 bedeutet R² einen aliphatischen oder aromatischen Rest, R und R¹ sind wie vorstehend beschrieben definiert.

Es können auch mehrere Kondensationsprodukte (K) zur Synthese eingesetzt werden. Hierbei können einerseits mehrere Alkohole beziehungsweise mehrere Carbonate eingesetzt werden. Weiterhin lassen sich durch die Wahl des Verhältnisses der eingesetzten Alkohole und der Carbonate bzw. der Phosgene Mischungen verschiedener Kondensationsprodukte unterschiedlicher Struktur erhalten. Dies sei am Beispiel der Umsetzung eines Carbonates mit einem dreiwertigen Alkohol beispielhaft erläutert. Setzt man die Ausgangsprodukte im Verhältnis 1:1 ein, wie in (II) dargestellt, so erhält man ein Molekül XY₂. Setzt man die Ausgangsprodukte im Verhältnis 2.1 ein, wie in (IV) dargestellt, so erhält man ein Molekül X₂Y. Bei einem Verhältnis zwischen 1:1 und 2:1 erhält man eine Mischung von Molekülen XY₂ und X₂Y.

Die beispielhaft in den Formeln 1 - 5 beschriebenen einfachen Kondensationsprodukte (K) reagieren bevorzugt intermolekular unter Bildung von hochfunktionellen Polykondensationsprodukten, im folgenden Polykondensationsprodukte (P) genannt. Die Umsetzung zum Kondensationsprodukt (K) und zum Polykondensationsprodukt (P) erfolgt üblicherweise bei einer Temperatur von 0 bis 300 °C, bevorzugt 0 bis 250°C, besonders bevorzugt bei 60 bis 200°C und ganz besonders bevorzugt bei 60 bis 160°C in Substanz oder in Lösung. Dabei können allgemein alle Lösungsmittel verwendet werden, die gegenüber den jeweiligen Edukten inert sind. Bevorzugt verwendet werden organische Lösungsmittel, wie zum Beispiel Decan, Dodecan, Benzol, Toluol, Chlorbenzol, Xylol, Dimethylformamid, Dimethylacetamid oder Solventnaphtha.

In einer bevorzugten Ausführungsform wird die Kondensationsreaktion in Substanz durchgeführt. Der bei der Reaktion freiwerdende monofunktionelle Alkohol oder das Phenol ROH kann zur Beschleunigung der Reaktion aus dem Reaktionsgleichgewicht entfernt werden, zum Beispiel destillativ, gegebenenfalls bei vermindertem Druck.

Falls Abdestillieren vorgesehen ist, ist es regelmäßig empfehlenwert, solche Carbonate einzusetzen, welche bei der Umsetzung Alkohole oder Phenole ROH mit einem Siedepunkt von weniger als 140°C bei dem vorliegenden Druck freisetzen.

Zur Beschleunigung der Reaktion können auch Katalysatoren oder Katalysatorgemische zugegeben werden. Geeignete Katalysatoren sind Verbindungen, die Veresterungs- oder Umesterungsreaktionen katalysieren, zum Beispiel Alkalihydroxide, Alkalicarbonate, Alkalihydrogencarbonate, vorzugsweise des Natriums, Kaliums oder Cäsiums, tertiäre Amine, Guanidine, Ammoniumverbindungen, Phosphoniumverbindungen, Aluminium-, Zinn-, Zink, Titan-, Zirkon- oder Wismut-organische Verbindungen, weiterhin sogenannte Doppelmetallcyanid (DMC)-Katalysatoren, wie zum Beispiel in der DE 10138216 oder in der DE 10147712 beschrieben.

Vorzugsweise werden Kaliumhydroxid, Kaliumcarbonat, Kaliumhydrogencarbonat, Diazabicyclooctan (DABCO), Diazabicyclononen (DBN), Diazabicycloundecen (DBU), Imidazole, wie Imidazol, 1-Methylimidazol oder 1,2-Dimethylimidazol, Titan-tetrabutylat, Titantetraisopropylat, Dibutylzinnoxid, Dibutylzinn-dilaurat, Zinndioctoat, Zirkonacetylacetonat oder Gemische davon eingesetzt.

Die Zugabe des Katalysators erfolgt im allgemeinen in einer Menge von 50 bis 10000, bevorzugt von 100 bis 5000 Gew. ppm bezogen auf die Menge des eingesetzten Alkohols oder Alkoholgemisches.

Ferner ist es auch möglich, sowohl durch Zugabe des geeigneten Katalysators, als auch durch Wahl einer geeigneten Temperatur die intermolekulare Polykondensationsreaktion zu steuern. Weiterhin lässt sich über die Zusammensetzung der Ausgangskomponenten und über die Verweilzeit das mittlere Molekulargewicht des Polymeren (P) einstellen.

Die Kondensationsprodukte (K) bzw. die Polykondensationsprodukte (P), die bei erhöhter Temperatur hergestellt wurden, sind bei Raumtemperatur üblicherweise über einen längeren Zeitraum stabil.

Aufgrund der Beschaffenheit der Kondensationsprodukte (K) ist es möglich, daß aus der Kondensationsreaktion Polykondensationsprodukte (P) mit unterschiedlichen Strukturen resultieren können, die Verzweigungen, aber keine Vernetzungen aufweisen. Ferner weisen die Polykondensationsprodukte (P) im Idealfall entweder eine Carbonat- oder Carbamoylchloridgruppe als fokale Gruppe und mehr als zwei OH-Gruppen oder aber eine OH-Gruppe als fokale Gruppe und mehr als zwei Carbonat- oder Carbamoylchloridgruppen auf. Die Anzahl der reaktiven Gruppen ergibt sich dabei aus der Beschaffenheit der eingesetzten Kondensationsprodukte (K) und dem Polykondensationsgrad.

Beispielsweise kann ein Kondensationsprodukt (K) gemäß der allgemeinen Formel 2 durch dreifache intermolekulare Kondensation zu zwei verschiedenen Polykondensationsprodukten (P), die in den allgemeinen Formeln 6 und 7 wiedergegeben werden, reagieren.

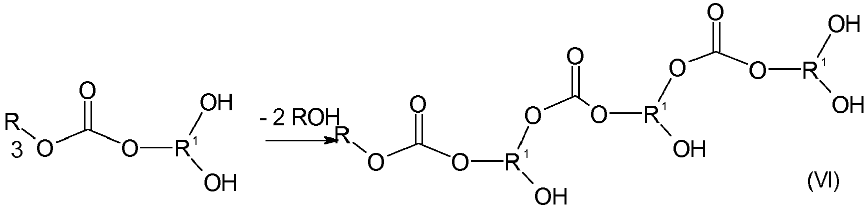

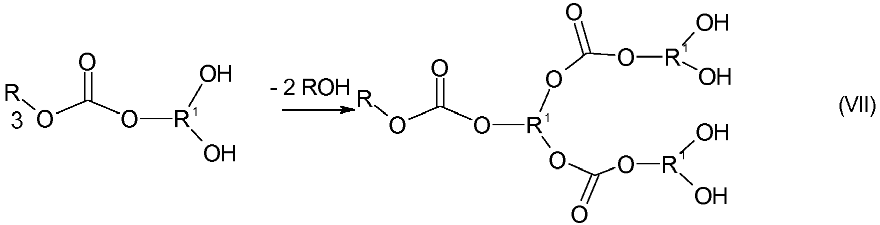

In Formel 6 und 7 sind R und R¹ wie vorstehend definiert.

Zum Abbruch der intermolekularen Polykondensationsreaktion gibt es verschiedene Möglichkeiten. Beispielsweise kann die Temperatur auf einen Bereich abgesenkt werden, in dem die Reaktion zum Stillstand kommt und das Produkt (K) oder das Polykondensationsprodukt (P) lagerstabil ist.

Weiterhin kann man den Katalysator desaktivieren, bei basischen Katalysatoren zum Beispiel durch Zugabe einer sauren Komponente, zum Beispiel einer Lewis-Säure oder einer organischen oder anorganischen Protonensäure.

In einer weiteren Ausführungsform kann, sobald aufgrund der intermolekularen Reaktion des Kondensationsproduktes (K) ein Polykondensationsprodukt (P) mit gewünschten Polykondensationsgrad vorliegt, dem Produkt (P) zum Abbruch der Reaktion ein Produkt mit gegenüber der fokalen Gruppe von (P) reaktiven Gruppen zugesetzt werden. So kann bei einer Carbonatgruppe als fokaler Gruppe zum Beispiel ein Mono-, Di- oder Polyamin zugegeben werden. Bei einer Hydroxylgruppe als fokaler Gruppe kann dem Produkt (P) beispielsweise ein Mono-, Di- oder Polyisocyanat, eine Epoxydgruppen enthaltende Verbindung oder ein mit OH-Gruppen reaktives Säurederivat zugegeben werden.

Die Herstellung der erfindungsgemäßen hochfunktionellen Polycarbonate erfolgt zumeist in einem Druckbereich von 0,1 mbar bis 20 bar, bevorzugt bei 1 mbar bis 5 bar, in Reaktoren oder Reaktorkaskaden, die im Batchbetrieb, halbkontinuierlich oder kontinuierlich betrieben werden.

Durch die vorgenannte Einstellung der Reaktionsbedingungen und gegebenenfalls durch die Wahl des geeigneten Lösemittels können die erfindungsgemäßen Produkte nach der Herstellung ohne weitere Reinigung weiterverarbeitet werden.

In einer weiteren bevorzugten Ausführungsform wird das Produkt gestrippt, dass heißt von niedermolekularen, flüchtigen Verbindungen befreit. Dazu kann nach Erreichen des gewünschten Umsatzgrades der Katalysator optional desaktiviert und die niedermolekularen flüchtigen Bestandteile, zum Beispiel Monoalkohole, Phenole, Carbonate, Chlorwasserstoff oder leichtflüchtige oligomere oder cyclische Verbindungen destillativ, gegebenenfalls unter Einleitung eines Gases, vorzugsweise Stickstoff, Kohlendioxid oder Luft, gegebenenfalls bei vermindertem Druck, entfernt werden.

In einer weiteren bevorzugten Ausführungsform können die erfindungsgemäßen Polycarbonate neben den bereits durch die Reaktion erhaltenden funktionellen Gruppen weitere funktionelle Gruppen erhalten. Die Funktionalisierung kann dabei während des Molekulargewichtsaufbaus oder auch nachträglich, d.h. nach Beendigung der eigentlichen Polykondensation erfolgen.

Gibt man vor oder während des Molekulargewichtsaufbaus Komponenten zu, die neben Hydroxyl- oder Carbonatgruppen weitere funktionelle Gruppen oder funktionelle Elemente besitzen, so erhält man ein Polycarbonat-Polymer mit statistisch verteilten von den Carbonat-, Carbamoylchlorid oder Hydroxylgruppen verschiedenen Funktionalitäten.

Derartige Effekte lassen sich zum Beispiel durch Zusatz von Verbindungen während der Polykondensation erzielen, die neben Hydroxylgruppen, Carbonatgruppen oder Carbamoylchloridgruppen weitere funktionelle Gruppen oder funktionelle Elemente, wie Mercaptogruppen, primäre, sekundäre oder tertiäre Aminogruppen, Ethergruppen, Carbonsäuregruppen oder deren Derivate, Sulfonsäuregruppen oder deren Derivate, Phosphonsäuregruppen oder deren Derivate, Silangruppen, Siloxangruppen, Arylreste oder langkettige Alkylreste tragen. Zur Modifikation mittels Carbamat-Gruppen lassen sich beispielsweise Ethanolamin, Propanolamin, Isopropanolamin, 2-(Butylamino)-ethanol, 2-(Cyclohexylamino)ethanol, 2-Amino-1-butanol, 2-(2'-Amino-ethoxy)ethanol oder höhere Alkoxylierungsprodukte des Ammoniaks, 4-Hydroxy-piperidin, 1-Hydroxy-ethylpiperazin, Diethanolamin, Dipropanolamin, Diisopropanol-amin, Tris(hydroxy-methyl)aminomethan,Tris(hydroxyethyl)aminomethan, Ethylendiamin, Propylendiamin, Hexamethylendiamin oder Isophorondiamin verwenden.

Für die Modifikation mit Mercaptogruppen lässt sich zum Beispiel Mercaptoethanol einsetzten. Tertiäre Aminogruppen lassen sich zum Beispiel durch Einbau von Triethanolamin, Tripropanolamin, N-Methyldiethanolamin, N-Methyldipropanolamin oder N,N-Dimethylethanolamin erzeugen. Ethergruppen können zum Beispiel durch Einkondensation von di- oder höherfunktionellen Polyetherolen generiert werden. Durch Zugabe von Dicarbonsäuren, Tricarbonsäuren, Dicarbonsäureestern, wie bespielsweise Terephthalsäuredimethylester oder Tricarbonsäureestern lassen sich Estergruppen erzeugen. Durch Reaktion mit langkettigen Alkanolen oder Alkandiolen lassen sich langkettige Alkylreste einbringen. Die Reaktion mit Alkyl- oder Aryldiisocyanaten generiert Alkyl-, Aryl- und Urethangruppen aufweisende Polycarbonate, die Zugabe von primären oder sekundären Aminen führt zur Einbringung von Urethan- oder Harnstoffgruppen.

In dieser Erfindung sind die hochverzweigten Polycarbonate ganz oder teilweise mit linearen oder verzweigten C₄- bis C₄₀-Alkyl-und/oder -Alkenylresten substituiert. Im Rahmen der vorliegenden Erfindung können Alkenylreste einfach oder mehrfach ungesättigt sein.

Substitution im Rahmen der vorliegenden Erfindung bedeutet, dass die hochverzweigten Polymere während und/oder nach der Polymerisationsreaktion mit Verbindungen A umgesetzt werden. Verbindungen A sind dadurch gekennzeichnet, dass sie einen linearen oder verzweigten C₄- bis C₄₀-Alkyl-und/oder Alkenylrest und eine reaktive Gruppe enthalten. Eine reaktive Gruppe der Verbindung A ist in der Lage, mit dem hochverzweigten Polymer zu reagieren. Bevorzugt enthalten Verbindungen A genau einen linearen oder verzweigten C₄- bis C₄₀-Alkyl-und/oder Alkenylrest und genau eine reaktive Gruppe.

Hochverzweigte Polymere, die mit Verbindungen A umgesetzt wurden, werden als substituierte hochverzweigte Polymere bezeichnet.

Die Substitution kann vollständig oder teilweise erfolgen. Das heißt im Fall der vollständigen Substitution, dass die reaktiven Gruppen des hochverzweigten Polymers vollständig mit Verbindungen A reagiert haben. Im Fall der teilweisen Substitution haben nicht alle reaktiven Gruppen des hochverzweigten Polymers mit Verbindungen A reagiert.

Bevorzugt sind die hochverzweigten Polymere mit Octyl- (Capryl-), Nonyl-, Decyl-(Caprinyl-), Undecyl-, Dodecyl- (Laurinyl-), Tetradecyl-, Hexadecyl- (Palmityl-), Heptadecyl-, Octadecyl- (Stearyl-) Resten und/oder den entsprechenden einfach oder mehrfach ungesättigten Äquivalenten, wie zum Beispiel mit Dodecenyl-, Hexadienyl- (Sorbinyl-), Octadecenyl-(Oleyl-), Linolyl- oder Linolenyl-Resten substituiert.

Unter Äquivalent ist in diesem Zusammenhang ein Kohlenwasserstoffrest zu verstehen, der sich von dem entsprechenden linearen oder verzweigten Alkylrest lediglich dadurch unterscheidet, dass er wenigstens eine Doppelbindung aufweist.

Die substituierten hochverzweigten Polycarbonate werden bevorzugt erhalten, indem das erhaltene hochfunktionelle, hoch- oder hyperverzweigte Polycarbonat mit einem geeigneten Funktionalisierungsreagenz, welches mit den OH- und/oder Carbonat- oder Carbamoylchlorid-Gruppen des Polycarbonates reagieren kann, umgesetzt wird.

Hydroxylgruppen enthaltende hochfunktionelle, hochverzweigte Polycarbonate können zum Beispiel durch Zugabe von Säurederivatgruppen wie Ester, Anhydride oder Amide oder Isocyanatgruppen enthaltenden Molekülen modifiziert werden. Beispielsweise lassen sich Säuregruppen enthaltende Polycarbonate durch Umsetzung mit Anhydridgruppen enthaltenden Verbindungen erhalten.

Dabei beträgt das molare Verhältnis der reaktiven Gruppen der Substitutionsverbindung zu den reaktiven Gruppen des hochverzweigten Polymers von 1:10 bis 1:1, bevorzugt von 1:5 bis 1:1,1, insbesondere bevorzugt von 1:2 bis 1:1,2. Ein besonders bevorzugter Bereich ist 1:1,7 bis 1:1,4.

In einer bevorzugten Ausführungsform der vorliegenden Erfindung erfolgt die Substitution mit einem Carbonsäurederivat der Formel R-CO-Y und/oder einem Isocyanat der Formel R-NCO, wobei die Reste die nachstehende Bedeutung haben.

R ist lineares oder verzweigtes C₄- bis C₄₀-Alkyl.

Y ist OR¹, OC(O)R² oder NR³₂. Dabei ist R¹ Wasserstoff oder lineares oder verzweigtes C₁- bis C₆-Alkyl, R² lineares oder verzweigtes C₄- bis C₄₀-Alkyl, wobei R und R² gleich oder verschieden sein können. R³ ist Wasserstoff oder lineares oder verzweigtes C₁-bis C₄-Alkyl, wobei die beiden Reste R³ gleich oder verschieden voneinander sein können.

Bevorzugte Verbindungen sind lineare C₄-C₄₀-Alkylisocyanate, besonders bevorzugt sind Octyl- (Capryl-) isocyanat, Nonylisocyanat, Decyl- (Caprinyl-) isocyanat, Undecylisocyanat, Dodecyl- (Laurinyl-) isocyanat, Tetradecylisocyanat, Hexadecyl- (Palmityl-) isocyanat, Heptadecylisocyanat, Octadecyl- (Stearyl-) isocyanat.

Weitere bevorzugte Verbindungen sind lineare C₄-C₄₀-Alkenylisocyanate mit einer oder mehreren Doppelbindungen, besonders bevorzugt sind Dodecenyl-, Hexadienyl- (Sorbinyl-), Octadecenyl-(Oleyl-), Linolyl- oder Linolenyl-isocyanat.

Eine ganz besonders bevorzugte Verbindung ist Stearylisocyanat.

Die Substitution kann beispielsweise in einem nachträglichen Verfahrensschritt (Schritt c)) erfolgen. Die Substitution kann aber auch bereits während der Herstellung der hochverzweigten Polymere erfolgen.

Bevorzugt erfolgt die Substitution in einem nachträglichen Verfahrensschritt.

Erfolgt die Substitution in einem nachfolgenden Verfahrensschritt, so wird bevorzugt das hochverzweigte Polycarbonat vorgelegt und eine oder mehrere Verbindungen A werden zugegeben.

Die Substitution erfolgt üblicherweise bei einer Temperatur von 0 bis 300 °C, bevorzugt 0 bis 250°C, besonders bevorzugt bei 60 bis 200°C und ganz besonders bevorzugt bei 60 bis 160°C in Substanz oder in Lösung. Dabei können allgemein alle Lösungsmittel verwendet werden, die gegenüber den jeweiligen Edukten inert sind. Bevorzugt verwendet werden organische Lösungsmittel, wie zum Beispiel Decan, Dodecan, Benzol, Toluol, Chlorbenzol, Xylol, Dimethylformamid, Dimethylacetamid oder Solventnaphtha. In einer bevorzugten Ausführungsform wird die Substitutionsreaktion in Substanz durchgeführt. Bei der Reaktion freiwerdende niedermolekulare Verbindungen können zur Beschleunigung der Reaktion aus dem Reaktionsgleichgewicht entfernt werden, zum Beispiel destillativ, gegebenenfalls bei vermindertem Druck.

Zur Vervollständigung der Reaktion kann es nötig sein, die Temperatur des Reaktionsbehälters nach der Zugabe der Verbindung A beziehungsweise, falls mehrere voneinander verschiedene Verbindungen A eingesetzt werden, nach der Zugabe der Verbindungen A anzuheben. Üblicherweise beträgt die Anhebung 10 bis 50°C, bevorzugt beträgt sie 20 bis 40°C.

Die Substitution der hochfunktionellen Polycarbonate erfolgt zumeist in einem Druckbereich von 0,1 mbar bis 20 bar, bevorzugt bei 1 mbar bis 5 bar, in Reaktoren oder Reaktorkaskaden, die im Batchbetrieb, halbkontinuierlich oder kontinuierlich betrieben werden.

Erfindungsgemäß ist die Verwendung eines substituierten hochverzweigten Polycarbonats als Verdickter in kosmetischen und/oder dermatologischen Formulierungen. Bevorzugt enthält die kosmetische Zusammensetzung mindestens einen kosmetischen geeigneten Träge.

Bevorzugt ist die Verwendung in hautkosmetischen Formulierungen. Bevorzugt wird die Verwendung als Ölverdicker.

### Hautkosmetische Zubereitungen

Die hautkosmetische Zusammensetzungen, insbesondere solche zur Pflege der Haut, können in verschiedenen Formen vorliegen und eingesetzt werden. So können sie z. B. eine Emulsion vom Typ Öl-in-Wasser (O/W) oder eine multiple Emulsion, beispielsweise vom Typ Wasser-in-Öl-in-Wasser (W/O/W). Auch emulgatorfreie Formulierungen wie Hydrodispersionen, Hydrogele oder eine Pickering-Emulsion sind vorteilhafte Ausführungsformen.

Die Konsistenz der Formulierungen kann von pastösen Formulierungen über fließfähige Formulierungen bis hin zu dünnflüssigen, sprühbaren Produkten reichen. Dementsprechend können Cremes, Lotionen oder Sprays formuliert werden. Zur Anwendung werden die erfindungsgemäßen kosmetischen Zusammensetzungen in der für Kosmetika und Dermatika üblichen Weise auf die Haut in ausreichender Menge aufgebracht. Der Salzgehalt der Hautoberfläche ist ausreichend, um die Viskosität der erfindungsgemäßen Zubereitungen derart zu erniedrigen, dass eine einfache Verteilung und Einarbeitung der Zubereitungen ermöglicht wird.

Die hautkosmetischen Zubereitungen liegen insbesondere als W/O- oder O/W-Hautcremes, Tag- und Nachtcremes, Augencremes, Gesichtscremes, Antifaltencremes, Mimikcremes, Feuchthaltecremes, Bleichcremes, Vitamincremes, Hautlotionen, Pflegelotionen und Feuchthaltelotionen vor.

Weitere vorteilhafte hautkosmetische Zubereitungen sind Gesichtswasser, Gesichtsmasken, Deodorantien und andere kosmetische Lotionen und Zubereitungen für die dekorative Kosmetik, beispielsweise Abdeckstifte, Theaterfarbe, Mascara, Lidschatten, Lippenstifte, Kajalstifte, Eyeliner, Makeups, Grundierungen, Rouges, Puder und Augenbrauenstifte.

Außerdem können die Zusammensetzungen verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Die hautkosmetischen Zubereitungen enthalten neben dem W/W-Emulsionspolymerisat und geeigneten Trägern noch weitere in der Kosmetik übliche Wirk- und/oder Hilfsstoffe, wie zuvor und nachfolgend beschrieben.

Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, natürliche und synthetische Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silikone, Feuchthaltemittel, Konditioniermittel, Rückfetter und weitere übliche Additive.

Den Zusammensetzungen können auch weitere Polymere zugesetzt werden, falls spezielle Eigenschaften eingestellt werden sollen. Zur Einstellung bestimmter Eigenschaften wie z. B. Verbesserung des Anfassgefühls, des Spreitverhaltens, der Wasserresistenz und/oder der Bindung von Wirk- und Hilfs-Stoffen wie Pigmenten, können die Zusammensetzungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane oder Silikonharze.

Weitere mögliche Inhaltsstoffe der Zusammensetzungen sind nachfolgend unter dem jeweiligen Stichwort beschrieben.

### Öle, Fette und Wachse

Die haut- und haarkosmetischen Zusammensetzungen enthalten bevorzugt auch Öle, Fette oder Wachse.

Bestandteile der Öl- und/oder Fettphase der kosmetischen Zusammensetzungen werden vorteilhaft gewählt aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Weitere polare Ölkomponenten können gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat Dicaprylyl Carbonat (Cetiol CC) und Cocoglyceride (Myritol 331), Butylen Glycol Dicaprylat/Dicaprat und Dibutyl Adipat sowie synthetische, halbsynthetische und natürliche Gemische solcher Ester, wie z.B. Jojobaöl.

Ferner können eine oder mehrere Olkomponenten vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Erfindungsgemäß vorteilhaft wird die Olkomponente gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether.

Erfindungsgemäß vorteilhaft sind Mischungen aus C12-C15-Alkylbenzoat und 2-Ethylhexylisostearat, Mischungen aus C12-C15-Alkylbenzoat und Isotridecylisononanoat sowie Mischungen aus C12-C15-Alkylbenzoat, 2-Ethylhexylisostearat und Isotridecylisononanoat.

Erfindungsgemäß besonders bevorzugt werden als Öle mit einer Polarität von 5 bis 50 mN/m Fettsäuretriglyceride, insbesondere Sojaöl und/oder Mandelöl eingesetzt.

Von den Kohlenwasserstoffen sind vorteilhaft Paraffinöl, Squalan, Squalen und insbesondere Polyisobutene, die auch hydriert sein können, im Sinne der vorliegenden Erfindung zu verwenden.

Ferner kann die Ölphase vorteilhaft gewählt werden aus der Gruppe der Guerbetalkohole. Guerbetalkohole entstehen nach der Reaktionsgleichung 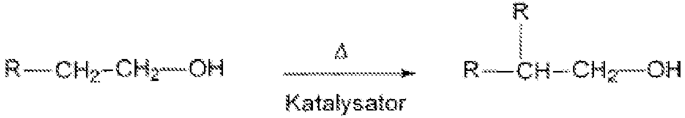 durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in kosmetischen Zusammensetzungen eingesetzt werden.

Die Verwendung von Guerbet-Alkoholen in Kosmetika ist an sich bekannt. Solche Species zeichnen sich dann meistens durch die Struktur 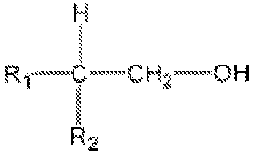 aus. Dabei bedeuten R₁ und R₂ in der Regel unverzweigte Alkylreste. Erfindungsgemäß vorteilhaft werden der oder die Guerbet-Alkohole gewählt aus der Gruppe, wobei
R₁ = Propyl, Butyl, Pentyl, Hexyl, Heptyl oder Octyl und
R₂ = Hexyl, Heptyl, Octyl, Nonyl, Decyl, Undecyl, Dodecyl, Tridecyl oder Tetradecyl.

Erfindungsgemäß bevorzugte Guerbet-Alkohole sind 2-Butyloctanol (beispielsweise als Isofol^{®}12 (Condea) kommerziell erhältlich) und 2-Hexyldecanol (beispielsweise als Isofol^{®}16 (Condea) kommerziell erhältlich).

Auch Mischungen von erfindungsgemäßen Guerbet-Alkoholen sind erfindungsgemäß vorteilhaft zu verwenden wie beispielsweise Mischungen aus 2-Butyloctanol und 2-Hexyldecanol (beispielsweise als Isofol^{®}14 (Condea) kommerziell erhältlich).

Auch beliebige Abmischungen solcher Öl- und Wachskomponenten sind vorteilhaft im Sinne der vorliegenden Erfindung einzusetzen. Unter den Polyolefinen sind Polydecene die bevorzugten Substanzen.

Vorteilhaft kann die Ölkomponente ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Niedermolekulare Silicone oder Siliconöle sind in der Regel durch folgende allgemeine Formel definiert 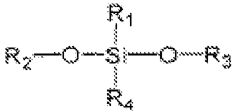 Höhermolekulare Silicone oder Siliconöle sind in der Regel durch folgende allgemeine Formel definiert 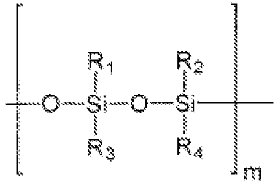 wobei die Siliciumatome mit gleichen oder unterschiedlichen Alkylresten und/oder Arylresten substituiert sein können, welche hier verallgemeinernd durch die Reste R₁ bis R₄ dargestellt sind. Die Anzahl der unterschiedlichen Reste ist aber nicht notwendigerweise auf bis zu 4 beschränkt. m kann dabei Werte von 2 bis 200.000 annehmen.

Erfindungsgemäß vorteilhaft einzusetzende cyclische Silicone sind in der Regel durch folgende allgemeine Formel definiert 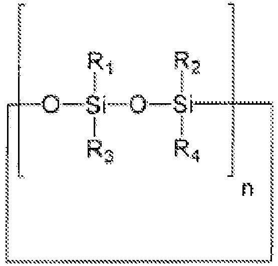 wobei die Siliciumatome mit gleichen oder unterschiedlichen Alkylresten und/oder Arylresten substituiert werden können, welche hier verallgemeinernd durch die Reste R₁ bis R₄ dargestellt sind. Die Anzahl der unterschiedlichen Reste ist aber nicht notwendigerweise auf bis zu 4 beschränkt. n kann dabei Werte von 3/2 bis 20 annehmen. Gebrochene Werte für n berücksichtigen, daß ungeradzahlige Anzahlen von Siloxylgruppen im Zyklus vorhanden sein können.

Vorteilhaft wird Phenyltrimethicon als Siliconöl gewählt. Auch andere Silikonöle, beispielsweise Dimethicon, Hexamethylcyclotrisiloxan, Phenyldimethicon, Cyclomethicon (z.B. Decamethylcyclopentasiloxan), Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden. Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxanpolyalkyl-Polyether-copolymere wie z.B. Cetyl-Dimethicon-Copolyol. Vorteilhaft wird Cyclomethicon (Octamethylcyclotetrasiloxan) als erfindungsgemäß zu verwendendes Silikonöl eingesetzt.

Vorteilhaft zu verwendende Fett- und/oder Wachskomponenten können aus der Gruppe der pflanzlichen Wachse, tierischen Wachse, Mineralwachse und petrochemischen Wachse gewählt werden. Vorteilhaft sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise Syncrowax^{®}HRC (Glyceryltribehenat), und Syncrowax^{®}AW 1 C (C₁₈₋₃₆-Fettsäure) sowie Montanesterwachse, Sasolwachse, hydrierte Jojobawachse, synthetische oder modifizierte Bienenwachse (z. B. Dimethicon Copolyol Bienenwachs und/oder C₃₀₋₅₀-Alkyl Bienenwachs), Cetyl Ricinoleate wie beispielsweise Tegosoft^{®}CR, Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride wie beispielsweise Hydriertes Soy Glycerid, Trihydroxystearin, Fettsäuren, Fettsäureester und Gly kolester wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan. Erfindungsgemäß können die Fett- und/oder Wachskomponenten sowohl einzeln als auch als Gemisch in den Zusammensetzungen verwendet werden.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, C₁₂₋₁₅-Alkylbenzoat, Capryl-Caprin-säure-triglycerid, Dicaprylylether. Besonders vorteilhaft sind Mischungen aus Octyldodecanol, Capryl-Caprinsäuretriglycerid, Dicaprylylether, Dicaprylyl Carbonat, Cocoglyceriden oder Mischungen aus C₁₂₋₁₅-Alkylbenzoat und 2-Ethylhexylisostearat, Mischungen aus C₁₂₋₁₅-Alkylbenzoat und Butylen Glycol Dicaprylat/Dicaprat sowie Mischungen aus C₁₂₋₁₅-Alkylbenzoat, 2-Ethylhexylisostearat und Isotridecylisononanoat.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden.

Die Ölkomponente kann auch vorteilhaft aus der Gruppe der Phospholipide gewählt werden. Die Phospholipide sind Phosphorsäureester acylierter Glycerine. Von größter Bedeutung unter den Phosphatidylcholinen sind beispielsweise die Lecithine, welche sich durch die allgemeine Struktur 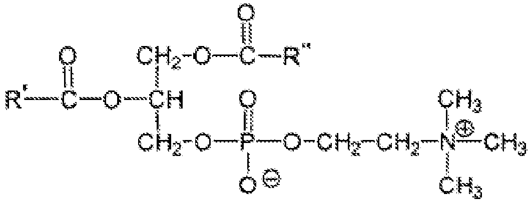 auszeichnen, wobei R' und R" typischerweise unverzweigte aliphatische Reste mit 15 oder 17 Kohlenstoffatomen und bis zu 4 cis-Doppelbindungen darstellen.

Als erfindungsgemäß vorteilhaftes Paraffinöl kann erfindungsgemäß Merkur Weissoel Pharma 40 von Merkur Vaseline, Shell Ondina^{®} 917, Shell OndinaK^{®} 927, Shell

Oil 4222, Shell Ondina^{®}933 von Shell & DEA Oil, Pionier^{®} 6301 S, Pionier^{®} 2071 (Hansen & Rosenthal) eingesetzt werden.

Geeignete kosmetisch verträgliche Öl- und Fettkomponenten sind in Karl-Heinz Schrader, Grundlagen und Rezepturen der Kosmetika, 2. Auflage, Verlag Hüthig, Heidelberg, S. 319 - 355 beschrieben, worauf hier in vollem Umfang Bezug genommen wird.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstands nicht nur in der jeweils angegebenen Kombination, sondern auch in anderen Kombinationen verwendbar sind, ohne den Rahmen der Erfindung zu verlassen.

Die vorliegende Erfindung soll anhand der nachfolgenden Beispiele näher erläutert werden.

### Beispiele

### Messmethoden

Die IR-Messungen erfolgten mit einem Nicolet 210-Gerät.
Die Bestimmung der Hydroxylzahl erfolgte nach DIN 53240, Teil 2.
Die Bestimmung desMolekulargewichts erfolgte mit Hilfe von Gelpermeationschromatographie mit einem Refraktometer als Detektor. Als mobile Phase wurde Dimethylacetamid verwendet, als Standard zur Bestimmung des Molekulargewichts wurde Polymethylmethacrylat (PMMA) eingesetzt.

### Einsatzstoffe

DBTL: Dibutylzinn-dilaurat, Hersteller: Fa. Sigma-Aldrich
Aluminiumchlorohydrat: Aktiviertes Aloxicoll^{®} Pulver. Hersteller: Fa. Giulini, Ludwigshafen, Deutschland
Hydrogeniertes Polyisobuten: Luvitol^{®} Lite, Hersteller: BASF Aktiengesellschaft, Ludwigshafen, Deutschland
Paraffinöl: Nujol, Fluka AG

### Beispiel 1: Herstellung eines hochverzweigten Polycarbonats

88,6 g Diethylcarbonat (0,75 mol) und 150 g (0,75 mol) eines Triols auf Basis Trimethylolpropan, das statistisch mit 1,2 Propylenoxideinheiten verethert wurde, wurden in einem 500-mL-Glasreaktor, ausgestattet mit Rührer, Rückflusskühler, Gaseinlass, anschließender Kühlfalle und Innenthermometer vorgelegt. Nach Zugabe von 0,02 g Kaliumcarbonat wurde die Mischung auf 120°C erwärmt, und 3 h bei dieser Temperatur gerührt. Der entstehende Ethanol wurde abdestilliert (46 g). Nach Beendigung der Destillation wurden 0,01 g Phosphorsäure zur Neutralisation des Katalysators zugegeben und es wurde eine Stunde bei 100°C gerührt. Anschließend wurde die Temperatur des Reaktionsgemisches auf 120°C erhöht und der restliche Ethanol unter einem Stickstoffstrom abgestrippt.

Das Endprodukt wurde über einen 125-µm-Filter filtriert und als klare, farblose niedrigviskoses Harz erhalten, das folgende Eigenschaften aufweist: Hydroxylzahl = 451 mg KOH/g; Mn=1300 g/mol, Mw=2000 g/mol

### Beispiele 2 - 9: Modifizierung des hochverzweigten Polycarbonats mit Stearylisocyanat

Hochverzweigtes Polycarbonat aus Beispiel 1 wurde in einem 250-mL-Glasreaktor, ausgestattet mit Rührer, Rückflusskühler, Gaseinlass, Innenthermometer und Tropftrichter, der die benötigte Menge Stearylisocyanat enthielt, vorgelegt. Die verwendeten Mengen an hochverzweigtem Polycarbonat und Stearylisocyanat sind der nachfolgenden Tabelle zu entnehmen.

Der Reaktor wurde auf 100°C erwärmt und das Isocyanat wurde innerhalb von 15 Minuten zugetropft. Danach wurde die Reaktionsmischung weitere drei Stunden bei 130°C gerührt und der Reaktionsfortschritt durch das Verschwinden der Isocyanatgruppen mit Hilfe von IR-Spektroskopie verfolgt (Schwingung der Isocyanat-Bande bei 2270 cm⁻¹).

| Beispiel | 2 | 3 | 4 | 5 | 6 | 7 | 8 | 9 |
|---|---|---|---|---|---|---|---|---|
| Hochverzweigtes Polycarbonat (Beispiel 1) [g] | 100 | 100 | 100 | 76 | 50 | 50 | 50 | 50 |
| Mol% NCO | 10 | 20 | 30 | 50 | 60 | 70 | 90 | 100 |
| Menge Stearylisocyanat [g] | 23,8 | 47,6 | 71,4 | 90,1 | 71,4 | 83,3 | 107,1 | 119 |

### Beispiel 10: Gelbildung durch Zugabe der Stearyl-modifizierten hochverzweigten Polycarbonate zu Paraffinöl

Verschiedene Mengen (0,5 bis 40 Gewichts-%) der Polymere der Beispiel 2 bis 9 wurden in Paraffinöl gelöst. Die Konzentration, bei der eine sichtbare Gelbildung eintrat, wird in der folgenden Tabelle angegeben:

| Polymer aus Beispiel ... | 2 | 3 | 4 | 5 | 6 | 7 | 8 | 9 |
|---|---|---|---|---|---|---|---|---|
| Verhältnis NCO/OH [%] | 10 | 20 | 30 | 50 | 60 | 70 | 90 | 100 |
| Gelbildungskonzentration (%) | 30 | 10 | 5 | 1,5 | 1 | 1 | 2,5 | 5 |

### Beispiel 11: Herstellung eines Deo-Sticks auf Basis von hydrogeniertem Polyisobuten-Öl

4 g des Polycarbonats aus Beispiel 7 and 20 g Aluminiumchlorhydrat werden mit 76 g hydrogeniertem Polyisobuten-Öl bei 80°C unter Rühren vermischt. Nach vollständigem Lösen des Polycarbonats wird die Mischung in eine Deostick-Form gefüllt und auf Umgebungstemperatur abgekühlt. Der fertige Deostick ist ein wachsartiges, bei Umgebungstemperatur festes Produkt..

### Beispiel 12: Herstellung eines Deo-Sticks auf Basis von Paraffinöl

4 g des Polycarbonats aus Beispiel 7 and 20 g Aluminiumchlorhydrat werden mit 76 g Paraffinöl bei 80°C unter Rühren vermischt. Nach vollständigem Lösen des Polycarbonats wird die Mischung in eine Deostick-Form gefüllt und auf Umgebungstemperatur abgekühlt. Der fertige Deostick ist ein wachsartiges, bei Umgebungstemperatur festes Produkt.Die Viskosität des Produkts beträgt 60 Pa s (20°C). Im Verlauf der Messung sinkt der Wert auf 30 Pa s.

## Patentansprüche

1. Verwendung eines substituierten hochverzweigten Polycarbonats, das ganz oder teilweise mit linearen oder verzweigten C₄- bis C₄₀-Alkyl- oder Alkenylresten substituiert ist in kosmetischen und/oder dermatologischen Formulierungen als Verdicker.

2. Verwendung nach Anspruch 1,
**dadurch gekennzeichnet, dass** die Substitution mit einem Derivat der Formel R-CO-Y und/oder R-NCO erfolgt
mit
R = lineares oder verzweigtes C₄- bis C₄₀-Alkyl,
Y = OR¹, OC(O)R², NR³₂, Halogen
R¹ = Wasserstoff, lineares oder verzweigtes C₁- bis C₆-Alkyl,
R² = lineares oder verzweigtes C₄- bis C₄₀-Alkyl, wobei R und R² gleich oder verschieden sein können,
R³ = Wasserstoff, lineares oder verzweigtes C₁- bis C₄-Alkyl, wobei die beiden Reste R³ gleich oder verschieden voneinander sein können.

3. Verwendung nach Anspruch 1 und/oder 2 in hautkosmetischen Formulierungen.

4. Verwendung nach einem der Ansprüche 1 bis 3 als Ölverdicker.

5. Verwendung nach Anspruch 1 oder 2, **dadurch gekennzeichnet, dass** die kosmetische Formulierung mindestens einen kosmetisch geeigneten Träger enthält.

## Claims

1. The use of a substituted highly-branched polycarbonate which is substituted completely or partly with linear or branched C₄-C₄₀-alkyl or alkenyl radicals, in cosmetic and/or dermatological formulations as thickeners.

2. The use according to claim 1,
wherein the substitution takes place with a derivative of the formula R-CO-Y and/or R-NCO
where
R = linear or branched C₄-C₄₀-alkyl,
Y = OR¹, OC(O)R², NR³₂, halogen
R¹ = hydrogen, linear or branched C₁-C₆-alkyl,
R² = linear or branched C₄-C₄₀-alkyl, where R and R² may be identical or different,
R³ = hydrogen, linear or branched C₁-C₄-alkyl, where the two radicals R³ may be identical or different from one another.

3. The use according to claim 1 and/or 2 in skin cosmetic formulations.

4. The use according to any one of claims 1 to 3 as oil thickener.

5. The use according to claim 1 or 2, wherein the cosmetic formulation comprises at least one cosmetically suitable carrier.

## Revendications

1. Utilisation d'un polycarbonate hautement ramifié substitué, qui est substitué en totalité ou en partie avec des radicaux alkyle ou alcényle en C₄ à C₄₀ linéaires ou ramifiés, dans des formulations cosmétiques et/ou dermatologiques en tant qu'épaississant.

2. Utilisation selon la revendication 1, **caractérisée en ce que** la substitution a lieu avec un dérivé de formule R-CO-Y et/ou R-NCO, avec
R = alkyle en C₄ à C₄₀ linéaire ou ramifié,
Y = OR¹, OC(O)R², NR³₂, halogène,
R¹ = hydrogène, alkyle en C₁ à C₆ linéaire ou ramifié,
R² = alkyle en C₄ à C₄₀ linéaire ou ramifié, R et R² pouvant être identiques ou différents,
R³ = hydrogène, alkyle en C₁ à C₄ linéaire ou ramifié, les deux radicaux R³ pouvant être identiques ou différents l'un de l'autre.

3. Utilisation selon la revendication 1 et/ou 2 dans des formulations cosmétiques pour la peau.

4. Utilisation selon l'une quelconque des revendications 1 à 3 en tant qu'épaississant huileux.

5. Utilisation selon la revendication 1 ou 2, **caractérisée en ce que** la formulation cosmétique contient au moins un véhicule cosmétiquement approprié.
